# FASCICULE DE BREVET EUROPEEN

(11) **EP 3 380 828 B1**
(45) Date de publication et mention de la délivrance du brevet: **06.11.2019**
(21) Numéro de dépôt: 16815603.2
(22) Date de dépôt: 25.11.2016
(51) Int. Cl.: G01N 21/65, G01N 33/569, C12Q 1/18

(54) **PROCÉDÉ DE DÉTERMINATION DE LA RÉACTION D'UN MICROORGANISME À SON EXPOSITION À UN COMPOSÉ CHIMIQUE**
VERFAHREN ZUR BESTIMMUNG DER REAKTION EINES MIKROORGANISMUS AUF SEINE EXPOSITION GEGENÜBER EINER CHEMISCHEN VERBINDUNG
METHOD FOR DETERMINING THE REACTION OF A MICROORGANISM TO ITS EXPOSURE TO A CHEMICAL COMPOUND

(30) Priorité: 27.11.2015 FR 1561446
(43) Date de publication de la demande: 03.10.2018
(73) Titulaire: Biomérieux, 69280 Marcy l'Étoile (FR); Commissariat à l'Énergie Atomique et aux Énergies Alternatives, 75015 Paris (FR)
(72) Inventeur: NOVELLI ROUSSEAU, Armelle, 38180 Seyssins (FR); ESPAGNON, Isabelle, 78470 Saint Remy les Chevreuse (FR); JOSSO, Quentin, 69007 Lyon (FR); DOUET, Alice, 16320 Villebois-Lavalette (FR); MALLARD, Frédéric, 38340 Voreppe (FR); GAL, Olivier, 92120 Montrouge (FR)
(74) Mandataire: Agasse, Stéphane
(86) Numéro de dépôt international: PCT/FR2016/053100
(87) Numéro de publication internationale: WO 2017/089727

(56) Documents cités:
- WO-A1-2017/089427
- WO-A2-03/042406
- US-A1- 2013 052 636
- TING-TING LIU ET AL: "A High Speed Detection Platform Based on Surface-Enhanced Raman Scattering for Monitoring Antibiotic-Induced Chemical Changes in Bacteria Cell Wall", PLOS ONE, vol. 4, no. 5, 7 mai 2009 (2009-05-07), pages e5470-e5470, XP055087067, ISSN: 1932-6203, DOI: 10.1371/journal.pone.0005470
- ASSMANN CORA ET AL: "Identification of vancomycin interaction withEnterococcus faecaliswithin 30 min of interaction time using Raman spectroscopy", ANALYTICAL AND BIOANALYTICAL CHEMISTRY, SPRINGER, DE, vol. 407, no. 27, 1 août 2015 (2015-08-01) , pages 8343-8352, XP035600866, ISSN: 1618-2642, DOI: 10.1007/S00216-015-8912-Y [extrait le 2015-08-01]
- KAI ZHAO ET AL: "Fabrication of silver-decorated sulfonated polystyrene microspheres for surface-enhanced Raman scattering and antibacterial applications", RSC ADV., vol. 5, no. 85, 10 août 2015 (2015-08-10), pages 69543-69554, XP055289596, DOI: 10.1039/C5RA11643K
- PAUL R. CAREY ET AL: "New techniques in antibiotic discovery and resistance: Raman spectroscopy", ANNALS OF THE NEW YORK ACADEMY OF SCIENCES, vol. 1354, 14 août 2015 (2015-08-14), pages 67-81, XP055289656, US ISSN: 0077-8923, DOI: 10.1111/nyas.12847
- UTE MÜNCHBERG ET AL: "Raman spectroscopic identification of single bacterial cells under antibiotic influence", ANALYTICAL AND BIOANALYTICAL CHEMISTRY, vol. 406, no. 13, 21 mars 2014 (2014-03-21), pages 3041-3050, XP055289930, DE ISSN: 1618-2642, DOI: 10.1007/s00216-014-7747-2
- A. I. M. ATHAMNEH ET AL: "Phenotypic Profiling of Antibiotic Response Signatures in Escherichia coli Using Raman Spectroscopy", ANTIMICROBIAL AGENTS AND CHEMOTHERAPY, vol. 58, no. 3, 2 décembre 2013 (2013-12-02), pages 1302-1314, XP055290095, US ISSN: 0066-4804, DOI: 10.1128/AAC.02098-13 cité dans la demande
- E. CONSUELO LÓPEZ-DÍEZ ET AL: "Monitoring the Mode of Action of Antibiotics Using Raman Spectroscopy: Investigating Subinhibitory Effects of Amikacin on Pseudomonas a eruginosa", ANALYTICAL CHEMISTRY, vol. 77, no. 9, 1 mai 2005 (2005-05-01), pages 2901-2906, XP055188604, ISSN: 0003-2700, DOI: 10.1021/ac048147m
- GYEONG BOK JUNG ET AL: "Evaluation of antibiotic effects on Pseudomonas aeruginosa biofilm using Raman spectroscopy and multivariate analysis", BIOMEDICAL OPTICS EXPRESS, vol. 5, no. 9, 28 août 2014 (2014-08-28), page 3238, XP055290203, United States ISSN: 2156-7085, DOI: 10.1364/BOE.5.003238

## Description

L'invention relève du champ de l'analyse du phénotype de sensibilité des microorganismes aux antibiotiques. Elle concerne la détermination de la réaction d'au moins un microorganisme d'intérêt à son exposition à un antibiotique mettant en œuvre une analyse par spectroscopie Raman et ses applications.

Le terme « microorganisme » couvre tout microorganisme susceptible de réagir à son exposition à un antibiotique, tel que les bactéries ou les levures. Bien que l'invention soit plus spécifiquement décrite ci-après en référence aux bactéries, il est entendu qu'elle ne s'y limite pas.

Cette détermination est d'intérêt majeur dans le diagnostic microbiologique dans les domaines de la santé, de l'agroalimentaire, de l'environnement, elle peut s'avérer tout aussi importante en pharmacologie, dans le criblage de nouvelles molécules, notamment antibiotiques, ou à la recherche de composés cytotoxiques présents dans des produits alimentaires, comme par exemple le lait. Cette sélection d'applications n'est pas exhaustive, et de manière générale, l'invention peut trouver une application dans tout domaine dès lors que la question est posée de la réaction de cellules à une exposition à un composé chimique ou biologique.

L'effet Raman est un phénomène de diffusion de la lumière qui s'applique à la très grande majorité des molécules. Son observation en spectroscopie permet de caractériser une molécule, un microorganisme, un milieu, elle est d'une mise en œuvre simple, elle est rapide et économique, et présente l'avantage substantiel en biologie de ne pas être fortement perturbée par l'eau et de ne pas nécessiter de marquage ou d'agent de contraste.

Ainsi selon le document AIM Athamneh et al. (2014) Antimicrob Agents Chemother 58:1302-1314, les auteurs ont utilisé la spectroscopie Raman afin de caractériser la sensibilité de cultures de *E. coli* à 15 antibiotiques connus représentant 5 familles d'antibiotiques, afin de constituer une base de référence. A cet effet, le procédé décrit comprend les étapes suivantes, réalisation d'une culture de *E. coli,* exposition d'un échantillon de cette culture à des antibiotiques en une concentration du triple de la concentration minimale inhibitrice (CMI), maintien desdites cellules en contact avec l'antibiotique pendant au moins 30 minutes, récolte et lavage des cellules bactériennes, prélèvement d'une suspension cellulaire et traitement pour analyse des couches de cellules par spectroscopie Raman. Le résultat de l'analyse est tiré de la moyenne d'une multiplicité de spectres obtenus pour chaque prélèvement comprenant une multitude de cellules et intégré dans la base de référence. Une fois constituée, cette base de référence peut être exploitée pour affecter un antibiotique inconnu à une desdites 5 familles, selon la sensibilité d'une culture de *E. coli* à cet antibiotique. Les performances de classification ainsi obtenues permettent d'obtenir des éléments quant à la classe de la molécule inconnue ce qui peut être utile pour la recherche pharmacologique. Les résultats obtenus ne fournissent toutefois pas d'information liée au phénotype de sensibilité des bactéries étudiées ni d'information propice à une utilisation clinique.

Le document WO2013/093913A1 décrit un procédé d'identification d'une bactérie dans un liquide biologique utilisant notamment la spectroscopie Raman. Un échantillon d'une culture bactérienne dudit liquide biologique est soumis à une lumière incidente et la lumière résultante obtenue par diffusion est analysée par spectroscopie Raman. Le signal lu est alors interprété grâce à une base de référence répertoriant la signature spectrale propre de différents microorganismes définie dans les mêmes conditions. Ce procédé peut en outre comprendre une étape d'exposition des dites bactéries à un antibiotique, le signal alors lu étant un effet dudit antibiotique sur ces bactéries. L'effet de l'antibiotique mesuré s'exerce notamment sur la viabilité des cellules bactériennes ou le développement de la culture. L'inconvénient de ce procédé réside dans son recours à une étape de culture qui, non seulement rallonge l'obtention d'une réponse, mais qui, de plus, nécessite de maîtriser une étape supplémentaire indispensable pour obtenir la réponse attendue.

Si l'utilisation de la spectroscopie Raman permet d'alléger la détermination du profil clinique de sensibilité à un antibiotique de bactéries d'intérêt, il n'en demeure pas moins que, selon cet art antérieur, elle est appliquée à une culture bactérienne dont l'obtention dans un temps de l'ordre 18 à 24 heures, ne permet pas d'accéder à un procédé de détermination rapide. En diagnostic, ceci constitue un frein important à une prise en charge efficace des patients.

Selon le document U. Münchberg et al. (2014) Anal Bioanal Chem 406 :3041-3050, les auteurs posent le problème de la mise en place rapide d'un traitement antibiotique approprié chez un patient, ainsi que les difficultés rencontrées par des techniques ayant recours à une culture cellulaire lorsque le patient a déjà reçu un traitement antibiotique. Les auteurs s'affranchissent alors de l'étape de culture et appliquent la spectroscopie Raman à des bactéries individuelles. Ces travaux abordent donc la question de la difficulté d'identification de bactéries ayant préalablement été exposées à un antibiotique, source potentielle d'un diagnostic erroné. Pour résoudre ce problème, les auteurs constituent une base de référence comprenant les résultats de l'analyse Raman réalisée sur des cellules individuelles non exposées à un antibiotique et sur des cellules qui ont été exposées à un antibiotique, dans différentes concentrations inférieures à la concentration minimale inhibitrice (CMI). La conclusion de cette étude est une absence de difficulté majeure à identifier les bactéries dans ces conditions. Les auteurs n'ont pas remarqué d'effet significatifs des antibiotiques sur les spectres bactériens et concluent que les modifications éventuelles sont observées dans des zones de fortes variabilité et donc inexploitables.

Le document WO 03/042406 A2 décrit un procédé permettant de caractériser le ou les mécanismes d'action d'antibiotiques sur des bactéries, mettant en œuvre une analyse par spectroscopie Raman.

Le document WO 2017/089427 A1 est compris dans l'état de la technique selon l'article 54(3) CBE.

Aucune de ces solutions ne permet d'envisager un procédé fiable de détermination du profil clinique de sensibilité à un antibiotique d'une bactérie d'intérêt et en particulier de sa résistance ou sa sensibilité à un antibiotique, en un temps court de l'ordre de quelques heures qui permettrait d'avoir un diagnostic dans la journée. Ce manque est responsable d'antibiothérapies inefficaces, de risques d'aggravation de l'infection du patient et d'une difficulté à rendre un diagnostic exact quand le patient a déjà été traité. Ce manque est d'autant plus ressenti à l'heure de l'émergence et la diffusion de bactéries multi-résistantes aux antibiotiques.

L'invention apporte une réponse à ce besoin avec un procédé de détermination de la réaction d'une souche bactérienne d'intérêt à son exposition à un antibiotique, ne nécessitant aucune culture, dont le résultat accessible en environ 2 heures est donc très rapide par rapport aux méthodes de l'état de l'art (36h-72h), et qui est en outre fiable.

L'invention concerne un procédé de détermination de la réaction d'au moins un microorganisme d'intérêt, tel qu'une bactérie d'intérêt, à son exposition à un antibiotique mettant en œuvre une analyse par spectroscopie Raman et comprenant les étapes suivantes :
On dispose d'un échantillon biologique susceptible de contenir lesdites bactéries d'intérêt,
On prépare au moins deux fractions dudit échantillon comprenant chacune une ou plusieurs bactéries d'intérêt vivantes,
On capture dans chaque fraction au moins une bactérie d'intérêt vivante à l'aide d'un partenaire de liaison,
On expose au moins l'une des fractions à au moins une concentration d'au moins un antibiotique donné, l'autre des fractions étant la fraction de contrôle,
On soumet la ou les bactérie(s) d'intérêt contenue(s)s dans les fractions à une lumière incidente et on analyse la lumière résultante obtenue par diffusion Raman par la ou les bactérie(s) d'intérêt par spectroscopie Raman pour obtenir autant de spectres Raman que de bactéries,
On soumet le matériau constituant le support dans les fractions à une lumière incidente et on analyse la lumière résultante obtenue par diffusion Raman dudit support par spectroscopie Raman pour obtenir quelques spectres Raman de ce support,
On traite lesdits spectres pour obtenir une signature de la réaction de la ou de chacune des bactérie(s) d'intérêt à l'exposition dudit antibiotique et du contrôle,
On compare la signature ainsi obtenue par bactérie d'intérêt, à une base de référence définie dans les mêmes conditions que ci-dessus, pour différentes bactéries et au moins ledit antibiotique, et
On définit un profil clinique de sensibilité de ladite bactérie d'intérêt audit antibiotique.

L'intérêt du procédé de l'invention par rapport à l'état de la technique précité, réside en ce qu'il permet d'obtenir un signal pertinent, corrélé aux modifications chimiques des microorganismes en réponse à leur exposition au composé testé à l'aide de la spectrométrie Raman, et ce pour des cellules individuelles. Non seulement il s'affranchit de l'étape de culture cellulaire, mais en outre, il fournit une information par cellule analysée. Contrairement aux procédés connus, l'information n'est pas obtenue par le calcul d'une moyenne des résultats d'analyse ou par l'obtention d'une mesure moyennée physiquement. Elle est l'expression d'un résultat par cellule, ce qui conduit à une information plus pertinente en ce qu'elle permet de détecter une variabilité, et qui pourrait être sensiblement différente de celle issue d'une moyenne de résultats qui masque toute hétérogénéité. Bien entendu, en fonction des applications considérées, l'information peut être obtenue à partir du résultat de plusieurs cellules individuelles.

Selon l'invention, on apporte un procédé de détermination de la réaction d'une bactérie à son exposition à un antibiotique, par spectroscopie Raman, qui est adapté à toutes les applications envisagées ci-dessus. Il peut effectivement être destiné à la caractérisation du profil clinique de sensibilité d'une bactérie d'intérêt à un antibiotique, dans un échantillon biologique, mais il peut en outre être aménagé pour le criblage de molécules antibiotiques. Puisqu'il n'a pas recours à une étape de culture, il est aussi approprié à l'analyse des cellules non cultivables.

Avant d'aborder plus en détails, la description du procédé de l'invention, certains termes employés sont ci-après définis.

Par échantillon biologique, on entend un tissu, un fluide, ainsi que des composants desdits tissu et fluide. Selon le domaine d'application du procédé, et à titre d'exemples non exhaustifs, l'échantillon peut être d'origine humaine ou animale tel que du sang, de l'urine, de la salive, du lait maternel ; il peut être d'origine végétale, être un extrait alimentaire, un extrait du sol...

Par réaction d'un microorganisme d'intérêt tel qu'une bactérie d'intérêt à son exposition à un antibiotique (ATB), on comprend toute modification, par exemple métabolique, qui peut être détectée par spectroscopie Raman par rapport à la même bactérie non exposée à un antibiotique.

La concentration minimale inhibitrice (CMI) d'un antibiotique pour une bactérie donnée, exprimée en µg/ml, est la plus petite concentration dudit antibiotique d'une gamme de dilutions capables de stopper la croissance bactérienne.

Un breakpoint clinique est une concentration donnée d'un antibiotique définie pour une espèce, déterminé par l'EUCAST (European Committee for Antimicrobial Susceptibility Testing) sur la base de critères microbiologiques et de données pharmacinétiques et pharmacodynamiques. Classiquement, deux breakpoints différents, dites valeurs seuil, sont définis et déterminent ainsi un intervalle de concentrations. Lorsque la CMI d'une souche testée est en-deçà de cet intervalle, la souche testée est qualifiée de sensible, c'est-à-dire qu'elle est susceptible d'être inhibée *in vivo* impliquant donc une forte probabilité de succès thérapeutique ; si elle se trouve dans cet intervalle, la bactérie est dite intermédiaire, et si elle est au-delà de cet intervalle, la bactérie est dite résistante, c'est-à-dire qu'elle supporte des concentrations d'antibiotique supérieures à celles acceptables *in vivo* et pour laquelle il existe une forte probabilité d'échec thérapeutique.

Par signature de la réaction d'un microorganisme d'intérêt à son exposition à un composé chimique, on entend toute variation par exemple métabolique ou constitutionnelle, exprimée par ledit microorganisme spécifiquement en réponse à son contact avec ledit composé et qui peut être détectée par spectrométrie Raman. Afin de mettre plus facilement en évidence ces variations, on peut également appeler signature le résultat d'une ou plusieurs étapes de traitement des données Raman aboutissant à un signal utilisé pour réaliser le test. A titre d'exemple, on peut choisir de soustraire un état initial ou une condition où les bactéries d'intérêt n'ont pas été exposées au composé testé aux spectres acquis sur des bactéries exposées.

Des variantes préférentielles d'un procédé de l'invention sont ci-après présentées, elles doivent être considérées seules ou en combinaison. Elles sont davantage appropriées à des applications diagnostiques, et précisément à la détermination du profil clinique de sensibilité d'une bactérie à un antibiotique dans un échantillon biologique, mais comme dit précédemment le procédé de l'invention n'est pas restreint à de telles applications.
La détermination du profil clinique de sensibilité d'une bactérie à un antibiotique consiste dans un premier temps à identifier le phénotype de sensibilité d'une bactérie à un antibiotique. Ainsi, on prépare de préférence plus de deux fractions dudit échantillon et on expose au moins deux fractions à des concentrations croissantes, respectivement, de l'antibiotique. Selon des variantes du procédé de l'invention, on expose au moins trois fractions dudit échantillon, voire quatre ou cinq ou plus, à des concentrations croissantes, respectivement, de l'antibiotique.

Avantageusement, les concentrations dudit antibiotique sont choisies dans un intervalle de valeurs reflétant des concentrations classiques de tests *in vitro,* permettant ainsi une comparaison aux données de référence actuelles, par exemple à aux tests par microdilution. Selon une variante préférentielle, les concentrations de l'antibiotique auxquelles la ou les fractions sont respectivement exposées, sont comprises dans un intervalle de valeurs incluant au moins l'une des valeurs choisies parmi les CMI typiques et les valeurs seuils des breakpoints cliniques pour le couple espèce/antibiotique testé, ou des panels de concentrations utilisés dans les méthodes de référence. Dans une application diagnostique du procédé de l'invention, ces concentrations sont donc spécifiquement choisies en fonction du couple bactérie/antibiotique considéré A titre d'exemple pour le couple *E.coli*/Gentamicine pour lesquelles la CMI typique, ou le cut-off épidémiologique de l'espèce, est de 2µg/mL et les deux breakpoints cliniques sont 2 et 4 µg/mL, elles peuvent être comprises dans un intervalle de valeurs incluant au moins l'une des valeurs choisies parmi 1, 2, 4 et 8 et 16 µg/ml, de préférence deux, voire trois ou quatre de ces valeurs ou même ces cinq valeurs.

Le procédé de l'invention comprend une étape de capture d'une bactérie d'intérêt au moyen d'un partenaire de liaison. Un partenaire de liaison selon l'invention reconnait spécifiquement ou non spécifiquement une bactérie d'intérêt pour la capture de celle-ci en vue de son analyse. Lors de son interaction avec la bactérie, le partenaire de liaison peut être présent à l'état libre dans le milieu ou peut avoir été préalablement immobilisé sur un support. Si la fixation de la bactérie sur le partenaire de liaison a lieu dans le milieu, une immobilisation dudit partenaire sur le support peut être réalisée ensuite. Par immobilisation sur un support, on entend une immobilisation directe ou indirecte dudit partenaire de liaison sur ledit support, par tout moyen bien connu de l'homme du métier. Un partenaire de liaison selon l'invention peut être de nature biologique et/ou chimique. Ainsi, à titre d'exemple, dans le cas d'une capture non spécifique, ou générique, des cellules, il peut consister en un composé chimique ou porter des fonctions chimiques qui interagira (ont) avec les cellules. Des polymères de type chitosan, poly-L-lysine, polyéthylènimine et poly-aniline en sont des illustrations. Il peut consister en une molécule biologique telle que choisie parmi les protéines, les anticorps, les antigènes, les aptamères, les phages, les protéines de phages ; ce sera généralement une capture spécifique des cellules. Dans une variante avantageuse de l'invention, le partenaire de liaison est immobilisé sur le support puis la bactérie capturée par ledit partenaire de liaison immobilisé.

L'étape de capture peut être effectuée sur des fractions d'une ou plusieurs bactéries exposées à un antibiotique et sur celle de contrôle, après concentration desdites fractions. Par exemple, on concentre lesdites fractions par centrifugation puis on récupère les culots que l'on soumet à l'étape de capture. De façon avantageuse, la capture est directement réalisée dans l'échantillon biologique sans étape séparée de concentration en culots.

Après l'étape de capture, on repère et on trie les bactéries capturées. Cette étape est réalisée par exemple par imagerie ou spectrophotométrie. Les bactéries non capturées peuvent alors être éliminées.

Les conditions d'exposition préférentielles des bactéries à l'antibiotique sont ci-après mentionnées :
L'antibiotique est dans un milieu physiologique permettant au moins de maintenir la ou les bactéries d'intérêt vivantes.

L'exposition à l'antibiotique est effectuée à une température de mise en culture des souches considérées, d'environ 18°C à environ 40°C, typiquement entre 28°C et 37°C pour les souches d'intérêt clinique.

L'exposition à l'antibiotique est effectuée pendant un temps dit d'incubation très inférieur au temps nécessaire dans les méthodes de référence. Selon l'invention, le temps d'incubation est avantageusement d'au moins 10 minutes et d'au plus 4 heures.

L'obtention d'une signature, pour chaque fraction exposée à l'antibiotique, sera illustrée dans les exemples.

De manière générale, plusieurs méthodes peuvent être utilisées pour traiter les données obtenues pour aboutir au résultat. Afin que celui-ci soit aussi pertinent que possible, une méthode complète de traitement des spectres au niveau individuel est de préférence réalisée. Elle est divisée en deux étapes majeures, une étape de prétraitement qui consiste à traiter les spectres pour maximiser l'extraction d'un signal d'intérêt et une étape de classification qui permet de réaliser le test d'intérêt proprement dit et aboutir au résultat d'intérêt.

L'étape de prétraitement comprend au moins l'une, de préférence deux, et mieux encore l'ensemble des opérations mentionnées ci-dessous :

### - Suppression des spectres saturés

La suppression des spectres saturés est la première étape de prétraitement des spectres. Elle est réalisée à partir des spectres bruts obtenus. On considère comme saturés les spectres pour lesquels plus de 20% des canaux de la région d'intérêt ont une intensité supérieure à 99% de l'intensité maximale.

### - Suppression des rayons cosmiques

Les rayons dits « cosmiques » sont des particules chargées de haute énergie, d'origine solaire, galactique ou extragalactique, qui bombardent en permanence le détecteur CCD (Charge-Coupled Device, dispositif à transfert de charge). Ils provoquent des pics de signal très fins qui peuvent apparaître de façon aléatoire dans les spectres. Une recherche de pics est d'abord réalisée à partir du calcul de la dérivée seconde du spectre brut. Une comparaison de cette dérivée seconde et de la dérivée seconde du spectre brut lissé permet ensuite d'identifier les pics très fins pour lesquels le lissage a diminué de façon significative la hauteur du pic, c'est-à-dire les rayons cosmiques. Les pics associés à des rayons cosmiques sont remplacés par une droite.

### - Réalignement

On a pu constater de légers décalages entre des séries de spectres prises à des dates différentes. Ce décalage est une constante sur l'ensemble du spectre. Il est préférable de le corriger.

La méthode consiste à réaligner tous les spectres par rapport à une « référence » constituée par les positions des 2 pics 1001 cm⁻¹ et 1126 cm⁻¹. La position des pics des spectres à réaligner est déterminée à partir d'un ajustement des pics par un modèle composé d'une gaussienne sur un fond affine.

Les spectres sur bactéries individuelles ne permettent généralement pas de réaliser un réalignement par spectre (spectres trop bruités). Le réalignement est donc de préférence effectué à partir des spectres moyens des bactéries (après suppression du fond par l'algorithme SNIP). Une comparaison entre la position de 2 pics dans le spectre net moyen à réaligner et les valeurs de référence de ces 2 mêmes pics permet de mesurer le décalage. On applique la correction trouvée à partir des spectres des bactéries aux spectres d'environnement de la même date acquis sur le matériau constituant le support.

### - Extraction du signal spécifique bactérien

Le fond est soustrait en deux étapes. Un premier fond, constitué d'un spectre moyen du matériau constituant le support, est ajusté par exemple entre 450 et 650 cm⁻¹ au spectre bactérie dans le cas d'un support en verre car c'est une région où il n'y a que la contribution spectrale du verre. Cet ajustement est réalisé avec la contrainte de rester sous le spectre bactérie dans cette région. La deuxième étape consiste à soustraire un fond par l'algorithme SNIP.

### - Suppression des déviants

Un module de suppression automatique des spectres déviants a été mis au point. Les spectres utilisés dans la recherche de déviants sont les spectres nets normalisés utilisés dans la suite de l'analyse. La recherche de déviants est appliquée à un groupe de spectres correspondant à une souche, une concentration d'antibiotique donnée et une date donnée. La méthode repose sur le calcul de la distance euclidienne entre chaque spectre et le spectre moyen d'un groupe de spectres. Cette suppression des déviants est réalisée deux fois consécutives. Le premier tour permet de supprimer les spectres très aberrants qui ont un effet non négligeable sur le spectre moyen.

### - Région d'intérêt et normalisation du signal

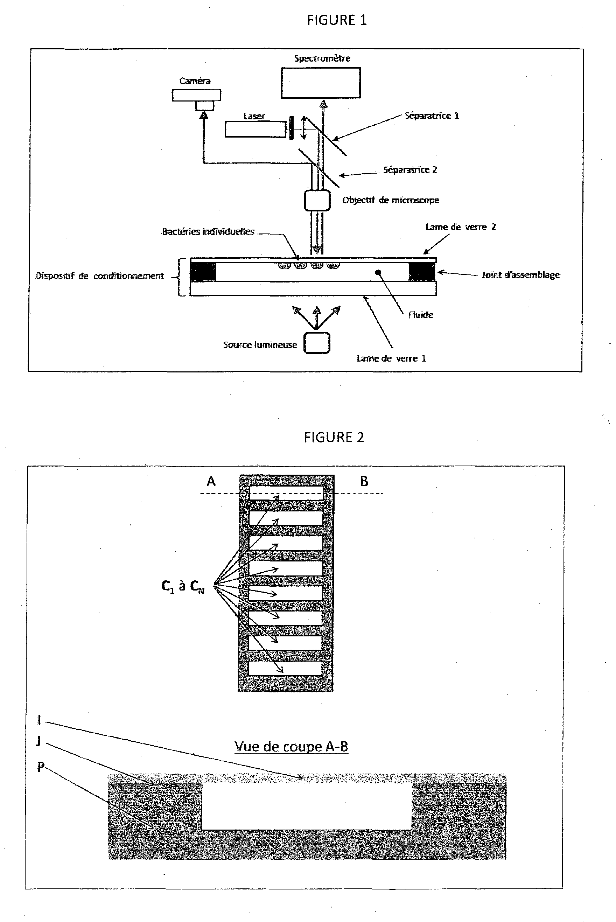

Le choix de la région d'intérêt est important car c'est sur cette région que les spectres seront comparés entre eux. Les spectres sont mesurés entre 400 et 3080 cm⁻¹ de décalage en énergie. La région d'intérêt retenue est [650-1750] cm⁻¹ et/ou [2800-3050] cm⁻¹. Il est indispensable de normaliser les signaux nets afin de pouvoir les comparer directement entre eux. L'intervalle de normalisation utilisé est [650-1750] cm⁻¹ ou [2800-3050] cm⁻¹. Le signal net est divisé par la valeur de la moyenne du signal net dans cet intervalle. Il est utile, dans un mode avantageux, de soustraire un état de référence des bactéries à l'ensemble des spectres de bactéries individuelles acquis. L'état de référence utilisé est celui constitué par des spectres issus de S₀. Cette opération permet de s'affranchir des variations des conditions de croissance, des variations de l'interface I (voir figure 2) et d'extraire un signal appelée signature lié à l'exposition à des concentrations d'agent antibiotique variables.

Comme indiqué précédemment, le procédé trouve un fort intérêt dans son application à la détermination du profil phénotypique clinique d'une bactérie d'intérêt et notamment pour déterminer sa sensibilité ou sa résistance à un antibiotique, et avantageusement déterminer la CMI de ladite bactérie audit antibiotique.

Dans cette indication et pour obtenir un résultat pertinent, on préférera que chaque fraction comprennent au moins 2, de préférence au moins 5, bactéries d'intérêt pour obtenir au moins 2, de préférence au moins 5 signaux.

Selon une variante de l'invention, les bactéries comparées sont sensiblement au même stade de croissance.

De manière générale, un procédé de l'invention peut être mis en œuvre dans un système comportant les éléments suivants :
- Un spectromètre permettant l'analyse Raman de l'échantillon :
   Le spectromètre Raman utilisé est classiquement qualifié de microspectromètre Raman confocal dans l'état de l'art en ce sens qu'il est constitué d'un étage d'analyse capable de produire un spectre à partir de la lumière résultante de la diffusion Raman après excitation par un laser, le spectromètre Raman, cet étage d'analyse étant couplé à un étage de microscopie confocale permettant de mesurer un spectre Raman à l'aide d'un objectif de microscope et de limiter le volume analysé à un volume restreint spatialement, le volume confocal. L'étage de microscopie du microspectromètre peut également permettre de façon classique l'acquisition d'images par une caméra présente dans le dispositif ou plus simplement par l'observation directe via des oculaires à l'aide d'une source lumineuse intégrée ou non au microspectromètre ;
- Un dispositif de conditionnement des micro-organismes pour l'analyse spectrale ; et
- Un ordinateur permettant le pilotage du microspectromètre, le stockage des données collectées et l'analyse de ces données à l'aide d'un logiciel dédié mettant en œuvre les méthodes ci-dessous.

Un dispositif de conditionnement des microorganismes tel que mentionné ci-dessus, éventuellement couplé à un spectromètre et à un ordinateur pour la mise en œuvre du procédé de l'invention est aussi un objet de l'invention.

Un système tel qu'abordé précédemment, permettant la mise en œuvre d'un procédé de l'invention est illustré à la figure 1 et sa mise en pratique est effectuée dans les exemples qui suivront. Ils sont ci-après décrits plus en détails :
Le système comprend un microspectromètre Raman permettant l'analyse confocale de la lumière diffusée par des objets de la taille des microorganismes (0.5-100 µm), par exemple un spectromètre Aramis de la marque HORIBA équipé d'un objectif de microscope ZEISS de type 100x Plan-Neofluar de référence 44080. Ce microspectromètre est équipé de moyens de visualisation manuels (oculaires) ou numériques (caméra CCD, par exemple de la marque IDS modèle µeye UI-1240ML) permettant l'observation des échantillons en position de mesure. Les paramètres de mesure Raman sont sélectionnés de façon appropriée pour l'objet étudié. Dans les exemples qui suivront, le volume confocal a été adapté pour être proche de la taille d'une bactérie (typiquement un trou confocal de 300 µm sur le modèle d'ARAMIS utilisé) afin de limiter les contributions spectrales non recherchées.
- Le système comprend aussi un dispositif de conditionnement, qui est un objet de l'invention, dont un mode de réalisation préféré **D** est illustré à la figure 2. Ce dispositif comporte :une pièce **P** comprenant des évidements correspondant à un ensemble de chambres (de **C₁** à **C_{N}**), N étant égal au moins à 2, lesdites chambres étant optionnellement isolables fluidiquement,
- un ensemble, optionnel, de ports **P₁** à **P_{2N}** permettant la connexion des chambres fluidiques à un système de gestion des liquides,
- une interface optique **I** fonctionnalisée ou non fonctionnalisée et compatible avec la mesure spectrale sur microorganismes ; et
- une partie **J,** optionnelle, assurant l'assemblage entre les pièces **I** et **P.**

Selon une variante simplifiée d'un dispositif de conditionnement de l'invention, il peut s'agir d'une lame de microscope standard I (25 mm x 75 mm x 1 mm, par exemple de la référence 631-1551 de chez VWR) constituant la pièce **P**, de deux adhésifs double-face faisant office de chambre fluidique (par exemple de la référence AB-0577 de chez Thermo Scientific couramment appelé « Gene Frame ») constituant le joint **J** et une lame couvre objet (par exemple de la référence 0107052 de chez Marienfeld) constituant l'interface **I**.

L'interface **I** peut être fonctionnalisée par une chimie de capture dite « générique » qui sera basée sur des propriétés généralement rencontrées chez les microorganismes en solution ou par une chimie de capture dite « spécifique » et basée sur des propriétés particulières d'une espèce recherchée. Par exemple la chimie de capture « générique » peut être matérialisée par l'absorption sur la lamelle couvre-objet de molécules polycationiques (comme le polyéthylènimine, la poly-L-lysine ou le chitosan...) et la chimie de capture spécifique peut être matérialisée par l'adsorption ou le couplage de molécules biologiques comme des protéines, des anticorps, des antigènes, des aptamères, des phages ou des protéines de phages, sur la surface de verre afin de permettre la capture d'un microorganisme d'intérêt.

Le dispositif de conditionnement assure les conditions physico-chimiques (températures, gaz...) permettant aux microorganismes d'intérêt de présenter une activité métabolique.

Des variantes de ce dispositif **D** sont bien entendu possibles et entrent dans le cadre de la présente invention.

Dans une mise en œuvre préférentielle, les étapes suivantes sont effectuées :
- on introduit une solution contenant les microorganismes d'intérêt dans les chambres C₁ à C_{N}, à l'aide des ports respectifs de connexion fluidique P₁ à P_{N},
- on respecte un temps dit de latence pour amener les microorganismes d'intérêt à se fixer à la surface **I** par interaction avec la fonctionnalisation,
- on introduit, respectivement, une série croissante de concentrations du composé chimique testé (C₂ à C_{N}) en solution liquide et une quantité prédéterminée de milieu physiologique dans chaque chambre fluidique C₁ à C_{N},
- on observe un temps dit d'incubation au cours duquel les microorganismes de chaque chambre sont exposés au composé chimique, à l'exception de ceux de la chambre C₁ (contrôle),
- on assure le repérage et le tri des microorganismes capturés sur la surface **I**,
- on réalise des mesures spectrales Raman sur les microorganismes isolés à l'étape précédente jusqu'à l'obtention d'un ensemble de spectres S₁ à S_{N} constitués des spectres acquis pour les microorganismes de chacune des chambres fluidiques C₁ à C_{N}, puis
- on effectue une analyse directe des spectres acquis, par comparaison de l'évolution des spectres de C₁ à C_{N} à une base de données préalablement constituée, ou par la recherche d'une signature spectrale en déterminant le jeu de spectres Sᵢ présentant une évolution significative,
- le résultat de l'analyse est constitué par la concentration Cᵢ, la comparaison de Cᵢ à un seuil de référence ou l'expression de l'évolution de certaines caractéristiques des spectres Sᵢ en fonction de la concentration ; il permet de qualifier le comportement phénotypique des microorganismes analysée par rapport au composé testé.

Comme indiqué précédemment et selon l'objectif recherché pour la mesure, plusieurs adaptations peuvent être réalisées (nombre de concentrations testées, temps d'incubation...), le principe général restant identique.

Les détails et avantages de l'invention ressortiront des exemples ci-après, à l'appui des figures suivants selon lesquelles :
Figure 1 représente le schéma de principe du montage complet d'un système permettant de mettre en œuvre le procédé de l'invention intégrant un dispositif de l'invention tel qu'illustré à la figure 2.
Figure 2 représente le schéma de principe du dispositif de conditionnement des microorganismes, appartenant au système illustré à la figure 1.
Figure 3 illustre une mise en place des échantillons sur une lamelle de verre du dispositif de conditionnement illustré à la figure 2.
Figure 4 représente une expression de la réaction de la souche d'Escherichia coli de référence ATCC 25922, appelée « EC10 », à la gentamicine, obtenue selon le procédé de l'invention.
Figure 5 représente une expression de la réaction de la souche d'Escherichia coli de référence ATCC 35421, appelée « EC21 », à la gentamicine, obtenue selon le procédé de l'invention, pour N=5.
Figure 6 représente la matrice de confusion obtenue pour la souche sensible d'Escherichia coli de référence ATCC 25922, appelée « EC10 », en présence d'amoxicilline (CMI = 6 µg/mL) pour N=11.
Figure 7 représente la matrice de confusion obtenue pour la souche sensible d'Escherichia coli de référence ATCC 35421, appelée « EC21 », en présence d'amoxicilline pour N=11.
Figure 8 représente la matrice de confusion obtenue pour la souche sensible d'Escherichia coli de référence ATCC 35421, appelées « EC21 », en présence d'amoxicilline testée avec un classifieur entrainé avec des bactéries exposées à de la gentamicine pour N=11.
Figure 9 illustre les signatures obtenues pour des concentrations de gentamicine de 0 µg/mL, 2 µg/mL et 8 µg/mL.
Figure 10 représente un schéma simplifié du principe proposé en exemple 4.
Figure 11 représente une expression de la réaction d'une souche bactérienne exposé à différentes concentrations de ciprofloxacine, obtenue selon le procédé de l'invention.
Figure 12 représente les matrices de confusion obtenues pour la souche sensible de Staphylococcus aureus de référence ATCC 25923, appelée « SA44 », en présence d'oxacilline (CMI = 0,25µg/mL) pour N=9 pour deux expériences, les bactéries étant obtenues à partir de cultures en milieu liquide.
Figure 13 représente la matrice de confusion obtenue pour la souche sensible de Staphylococcus aureus de référence ATCC 25923, appelée « SA44 », en présence d'oxacilline (CMI = 0,25µg/mL) pour N=9, les bactéries étant obtenues à partir de cultures en milieu gélosé en boîte de Petri.

### Exemple 1: Application du procédé de l'invention à la détermination du phénotype de sensibilité de la souche d'Escherichia coli de référence ATCC 25922 appelée « EC10 » à la gentamicine

Le dispositif de conditionnement retenu est constitué de deux chambres fluidiques et deux concentrations d'antibiotique sont testées: c₀ : « Sans antibiotique » et c₁ : « Test de résistance ».

Soit c₁, la concentration de la gentamicine : c₁ = 8 µg/mL, qui correspond au doublement de la concentration 4 µg/mL qui correspond au breakpoint clinique tel que défini par l'EUCAST. L'objectif de ce test est de déterminer si la bactérie est considérée comme résistante au sens des définitions proposées par l'EUCAST.

Une solution contenant les bactéries à tester est utilisée comme échantillon de test. Cette solution est obtenue par la mise en suspension de 5.10⁷CFU/mL afin de représenter une concentration potentiellement rencontrée dans un échantillon clinique, par exemple un prélèvement urinaire. Cette solution de bactéries est mise en contact avec l'interface **I** du dispositif fonctionnalisée par l'adsorption de polyéthylènimine (PEI) (capture générique). Après un temps de capture de 10 minutes permettant aux bactéries de rentrer en contact avec la fonctionnalisation, l'interface **I** subit un lavage avec une solution d'eau, cette étape optionnelle permettant d'éliminer le surplus de bactéries non capturées encore en solution. Le milieu physiologique retenu dans cet exemple est constitué d'un mélange peu riche de Bouillon TSB-T Trypcase Soja broth (par exemple de la référence 42100 de bioMérieux) et de PBS 10x (par exemple obtenu à partir des comprimés PBS de référence A9162, 0100 de la marque AppliChem) dans un rapport 1:9. Après division en deux fractions de ce milieu physiologique, on ajoute respectivement dans chacune de ces fractions une quantité de gentamicine (par exemple de la référence G1397-10ML de Sigma-Aldricht) permettant d'obtenir une concentration différente de gentamicine c₀ ou c₁. Les solutions de concentrations c₀ et c₁ ainsi produites sont respectivement introduites dans les chambres C₀ ou C₁. Les bactéries capturées directement à partir de l'échantillon sur la surface **I** sont ainsi exposées, dans un milieu adapté, à une concentration d'antibiotique différente selon la chambre dans laquelle elles sont présentes.

Le dispositif est ensuite chauffé pour atteindre une température de 37°C pendant deux heures puis placé en position de mesure sur le microspectromètre. Le repérage des bactéries capturées est effectué par une procédure automatique basée sur l'analyse de l'image, par une procédure classique de détection de particules, acquise au moyen de la caméra du microspectromètre et d'une source de lumière adaptée. Ce repérage permet d'acquérir automatiquement une série de spectres Raman (S₀ et S₁, respectivement) acquis sur des bactéries individuelles présentes dans chaque chambre C₀ et C₁. Le nombre de spectres à acquérir pour constituer un jeu de données dépend du niveau d'exigence sur les performances des tests à réaliser.

Comme dit précédemment, plusieurs méthodes peuvent être utilisées pour traiter les données obtenues pour aboutir au résultat. Dans le présent exemple, on utilise une méthode complète de traitement des spectres au niveau individuel comprenant une étape de prétraitement comprenant l'ensemble des phases décrites précédemment pour maximiser l'extraction d'un signal d'intérêt et la classification.

Dans cet exemple, on utilise un ensemble d'au moins 2N spectres extrait de l'ensemble M total des spectres acquis : N spectres issu de **S₀** et N spectres issus de **S₁**. Ces spectres sont tirés sans remise parmi les M spectres disponibles. On soustrait à chacun des N spectres issus de **S₁** et des N spectres issus de **S₀** la moyenne des N spectres issus de **S₀**, ces deux lots de spectres constituant un « échantillon de test de contrôle» et un « échantillon de test de résistance ».

Pour la classification, on utilise dans le présent exemple une base de données de référence obtenue à partir d'expériences similaires réalisées précédemment à des dates et à partir de cultures différentes pour entrainer un classifieur obtenu à l'aide d'un Machine à Vecteur de Support (SVM) à noyau radial. Ce classifieur est entrainé à reconnaitre deux classes, l'une « Sans effet antibiotique » à partir de spectres issus de conditions sans antibiotique et l'autre « Effet antibiotique » à partir de spectres préalablement acquis dans des conditions où la concentration est supérieure à la CMI de la ou des souches utilisées dans la base de référence. Pour chaque échantillon de tests constitués de N spectres de différence, on teste individuellement ces spectres de différence par rapport au classifieur et on attribue au groupe de N spectres la classe majoritaire parmi les éléments des groupes. Cette attribution à la majorité est basée sur la bonne corrélation des résultats ainsi obtenus avec les méthodes de référence mais peut tout à fait être modifiée pour prendre en compte certains autres paramètres des tests. Par exemple un vote à seuil différent de la majorité où dès que le nombre de bactéries ne présentant aucun effet dépasse les 30% alors on attribue de façon conservatrice un résultat « Sans effet antibiotique ». On pourra également ajuster ce seuil pour prendre en compte le temps d'incubation : ainsi par exemple si on réduit de façon notable le temps d'exposition aux antibiotiques, ou que le microorganisme testé présente un temps de doublement typique plus lent, prendre en compte un seuil plus bas pour attribuer un résultat « effet antibiotique » à ce groupe de spectres. Enfin on pourrait également adopter un système plus nuancé où chaque bactérie est considérée de façon totalement individuelle. Ce dernier mode de réalisation peut-être avantageux si la méthode de la présente invention est utilisée à des fins de recherche.

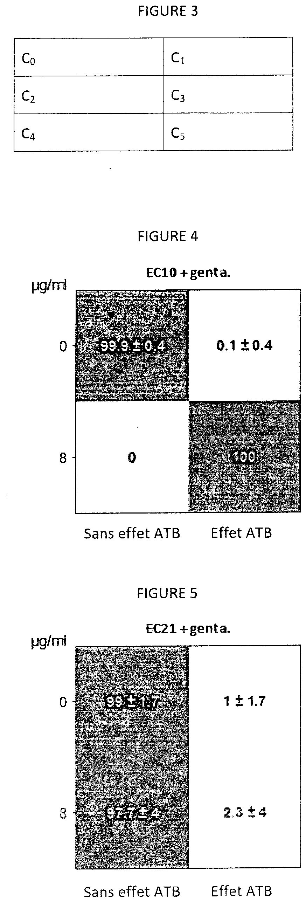

Afin d'illustrer les performances ainsi obtenues, le score moyen obtenu pour tous les résultats qui seraient obtenus avec une combinaison de 5 spectres par concentration (N=5) parmi un jeu total de spectres total acquis 294 spectres (M=294) est présenté à la figure 4. Cette matrice présente en colonne les états « Sans effet ATB » et « Effet ATB » et en ligne les deux concentrations d'antibiotiques testés. Le score indique le pourcentage des échantillons de test qui sont attribués à une classe donnée par le classifieur décrit précédemment. Ainsi on constate que 99% des échantillons de N=5 bactéries de l'« échantillon de test de contrôle» sont classés « Sans effet antibiotique » et que 97,1% des « échantillons de test de sensibilité » constitués par des bactéries exposées à la concentration de test sont classés « Effet ATB ». La souche peut donc être qualifiée de sensible selon l'invention avec une grande confiance sur la base de seulement quelques analyses de bactéries individuelles. Le résultat est conforme aux méthodes de référence [Produits bioMérieux ETEST® GM 256 (réf. 412368) et VITEK® carte N233 (réf. 413117)] qui donnent pour résultat une CMI=1µg/mL ce qui confirme bien que la bactérie n'est pas résistante au sens de l'EUCAST, sa CMI n'étant pas strictement supérieure au seuil défini par cet organisme.

### Exemple 2 : Application du procédé de l'invention à la détermination du phénotype de sensibilité de la souche d'Escherichia coli de référence ATCC 35421 appelée « EC21 » à la gentamicine

Un test identique à celui de l'exemple 1 est mené pour une autre souche d'*Escherichia coli,* la souche ATCC 35421 appelée « EC21 » permet de confirmer le caractère discriminant de la mesure.

Les résultats sont présentés à la figure 5.

Les méthodes de référence attribue une CMI>256µg/mL à cette souche qui est donc résistante au sens de l'EUCAST.

La méthode de l'invention confirme ce résultat puisque dans le cas N=5 et M=133, on lit que 100% des tests effectués ne présentent pas de profil caractéristique d'effet de l'agent antibiotique.

### Exemple 3 : Détermination de la concentration minimale inhibitrice (CMI) de deux souches d'Escherichia coli à l'amoxicilline

Dans cet exemple, on cherche à déterminer le phénotype de sensibilité, et à préciser un encadrement de la concentration minimale inhibitrice de la souche d'*Escherichia coli* de référence ATCC 25922 appelé « EC10 » pour un antibiotique, l'amoxicilline, possédant un mode d'action différent que celui proposé dans les exemples 1 et 2.

Afin d'illustrer le pouvoir discriminant de cette méthode, le même test est réalisé pour une souche résistante à l'amoxicilline d'*Esherichia coli* ATCC 35421 appelé « EC21 » est aussi présenté.

Les concentrations suivantes d'amoxicilline ont été testées.

EC10 souche sensible (CMI_{REF}=6µg/mL) :
0 µg/mL; 2 µg/mL ; 4 µg/mL ; 8 µg/mL et 16 µg/mL
EC21 souche résistante (CMI_{REF}=256µg/mL) :
0 µg/mL ; 4 µg/mL et 8 µg/mL.

Dans cet exemple, un autre mode de réalisation du test proposé correspondant à la méthode alternative décrite est illustré.

Une solution contenant les bactéries à tester, EC10 ou EC21, est utilisée comme échantillon de test. Cette solution est obtenue par la mise en suspension dans l'eau de 5 10⁷CFU/mL afin de représenter une concentration potentiellement rencontrée dans un échantillon clinique, par exemple un prélèvement urinaire. Cette solution de bactéries est répartie dans 5 tubes à filtre (par exemple Microcon YM100 de Millipore) à raison de 150µlL par tube. On ajoute à chacun de ces tubes une quantité suffisante pour 250µL finaux de milieu physiologique permettant la croissance constitué dans cet exemple d'un mélange de PBS de concentration finale 1x (obtenu à partir des comprimés PBS de référence A9162,0100 de la marque AppliChem), de milieu nutritif TSB 0.1X (par exemple obtenu à partir de Bouillon TSB-T Trypcase Soja broth de référence 42100 de bioMérieux) et d'une quantité d'amoxicilline permettant d'aboutir à des concentrations finales respectives c₀ à c₄ d'amoxicilline (par exemple la référence A8523-10ML de Sigma-Aldricht) comme suit :
- c₀=0µg/mL
- c₁=2µg/mL
- c₂=4µg/mL
- c₃=8µg/mL
- c₄=16µg/mL

Les 5 tubes ainsi obtenus sont incubés pendant 2 heures à 37°C sous agitation. Une centrifugation, à 1200g pendant 8 minutes à l'aide d'une centrifugeuse adaptée aux contenants utilisés (par exemple le modèle 8415C de la marque Eppendorf) permet alors de récupérer un culot bactérien sur la partie filtre de chaque tube et d'éliminer le milieu. Les culots bactériens sont respectivement resuspendus en eau pour effectuer un lavage avant d'être à nouveau culotés par centrifugation (1200g pendant 10 minutes) toujours sur la partie filtre du tube. Ces culots sont répartis sur une lame de verre de type Marienfield constituant l'interface **I** (non fonctionnalisé dans cette configuration) à l'aide d'un écouvillon dans des chambres correspondantes noté C₀ à C₄. Dans cette configuration, les chambres ne sont pas nécessairement isolées d'un point de vue physique puisqu'aucun échange n'est possible entre les différentes conditions. Il est ainsi possible d'utiliser une lamelle de verre dont des compartiments virtuels sont clairement identifiés pour chaque concentration tel que présenté à la figure 3. Les compartiments virtuels sont définis par des délimitations matérialisées dans le présent exemple par un marquage préalablement effectué sur la lamelle du côté opposé à celui où sont déposées les bactéries. La lame de verre est ensuite déposée sur un « geneframe » constituant le joint **J** dans le dispositif de conditionnement précédemment décrit.

Le repérage des bactéries capturées est effectué par une procédure manuelle dans cet exemple basée sur l'analyse visuelle par l'opérateur de l'image acquise au moyen de la caméra du microspectromètre et d'une source de lumière adaptée par un expérimentateur. Ce repérage permet d'acquérir une série d'au moins N spectres Raman acquis sur des bactéries individuelles présentes respectivement dans chaque chambre C₀ à C₄. Le nombre de spectres à acquérir pour constituer un jeu de données dépend du niveau d'exigence sur les performances des tests à réaliser.

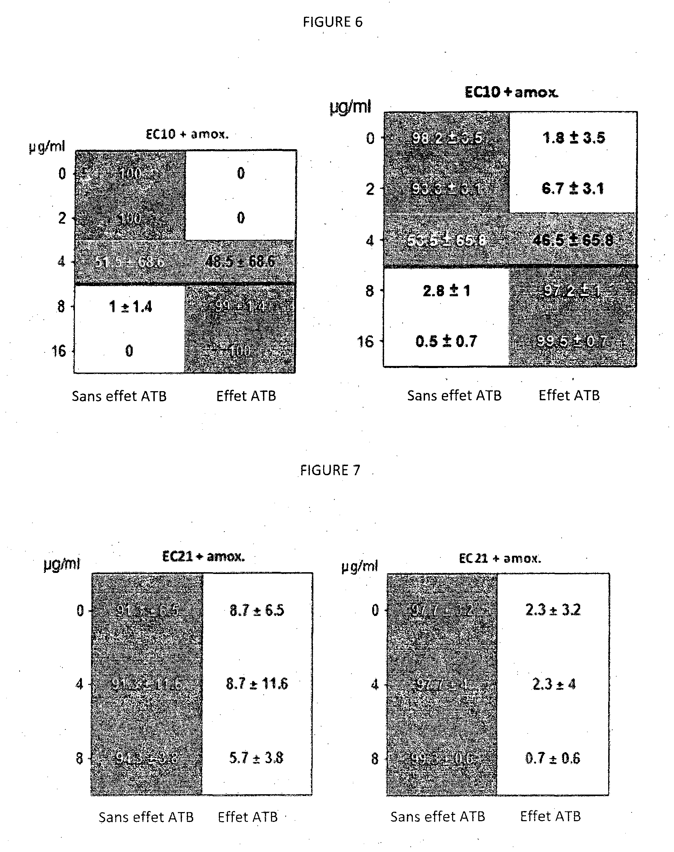

Le mode de traitement de données proposé ici est identique à celui des exemples 1 et 2 : une première étape de prétraitement suivie d'une étape de classification des spectres acquis à l'aide d'un classifieur préalablement entrainé. Dans les exemples proposés ci-dessous le classifieur est entrainé avec une base de référence contenant des spectres « Sans effet antibiotique » préalablement acquis dans une condition sans antibiotique (0µg/mL) d'amoxicilline de bactéries EC10 et de spectres « Effet antibiotique » de bactéries EC10 en présence de 8µg/mL d'amoxicilline. Les résultats obtenus sont présentés dans la matrice de confusion proposée en figure 6. Comme précédemment cette matrice permet de démontrer la robustesse de la méthode en donnant les résultats pour un grand nombre de tests.

On observe une transition dans l'attribution des groupes de spectres de la catégorie « Sans effet ATB » vers la catégorie « Effet antibiotique » entre les concentrations 4µg/mL et 8µg/mL. On peut donc attribuer à cette souche une CMI comprise entre 4µg/mL et 8µg/mL selon les essais. Cette variabilité à un facteur de dilution près est très fréquente dans ce type d'essai, l'EUCAST indique par exemple des gammes de variations de CMI de [2-8]µg/mL pour cette souche ATCC 25922 lors des contrôles qualité des tests de CMI en diffusion par disque, et est donc conforme avec des résultats attendus. Le résultat établi par les méthodes de référence [Produits bioMérieux ETEST® AM 256 (réf. 412253) et VITEK® carte N233 (réf. 413117)] est de 6µg/mL pour cette souche ce qui est également conforme avec ce résultat.

Le même type d'expérience réalisé sur la souche « EC21 » résistante à l'amoxicilline donne les résultats présentés à la figure 7. Aucune transition n'est observée et la très grande majorité des groupes mesurés est attribuée à la catégorie « Sans effet ATB ». On peut donc attribuer une CMI>8µg/mL à cette souche avec ce test ce qui est également conforme avec les résultats obtenus par la méthode de référence.

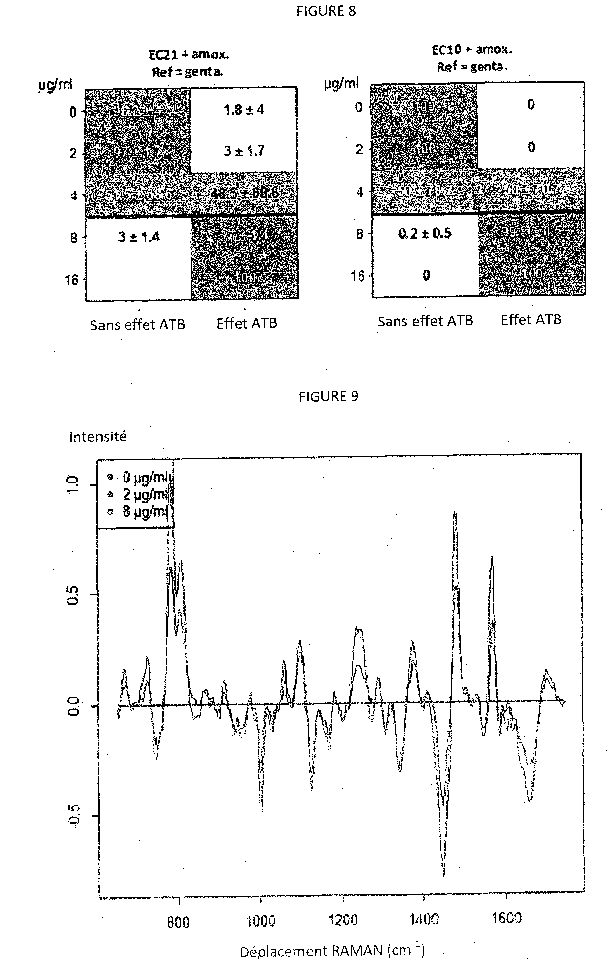

Comme le montre la figure 8, des résultats identiques sont obtenus en entrainant le classifieur sur une base de référence contenant à nouveau des spectres de bactéries non exposées à des antibiotiques pour reconnaitre la classe « Sans effet ATB » et des spectres de bactéries « Effet antibiotique » exposés à une concentration supérieure à la CMI d'une autre molécule antibiotique appartenant à une famille différente, par exemple la gentamicine de l'exemple précédent.

On retrouve des résultats similaires à ceux exposés précédemment. Cet exemple prouve qu'il est possible d'effectuer la recherche de l'effet antibiotique d'une substance inconnue sur la souche bactérienne testée de cette manière et pourrait donc être appliqué au criblage de molécule.

### Exemple 4 : Détermination de l'effet d'une substance inconnue sur une souche bactérienne.

Dans cet exemple, on cherche à déterminer le phénotype de sensibilité, et à préciser un encadrement de la concentration minimale inhibitrice, d'une souche bactérienne, par exemple, des souches d'*Escherichia coli* de référence ATCC 25922 appelée « EC10 », pour une substance réputée inconnue.

Pour les besoins du test, on utilise une molécule antibiotique connue mais n'appartenant pas aux familles antibiotiques précédemment utilisées : la ciprofloxacine de la famille des fluoroquinolones.

Le mode de réalisation de l'exemple précédent est utilisé pour cet exemple. Seuls les résultats obtenus pour les 4 premières concentrations seront explicitement illustrés car un effet antibiotique est rapidement détecté pour cette molécule. Une série de N spectres Raman est acquis dans chacune des chambres de C₀ à C₃. Les concentrations c₀ à c₃ utilisées sont les suivantes :
- C₀=0µg/mL
- c₁=0,005µg/mL
- c₂=0,015µg/mL
- c₃=0,064µg/mL

Comme précédemment, les étapes réalisées pour effectuer le prétraitement des spectres sont les suivantes :
- la suppression des spectres saturés
- la suppression des rayons cosmiques
- le réalignement
- l'extraction du signal spécifique bactérien
- la suppression des déviants
- la région d'intérêt et la normalisation du signal

Pour effectuer un test, on réalise une moyenne de N spectres acquis pour chaque concentration testée et on soustrait au résultat une moyenne de N spectres de la concentration c₀. Pour la concentration c₀ on choisit N spectres différents des N spectres utilisés pour la soustraction de l'état de référence. Cette opération a pour objectif de s'affranchir au maximum de toutes variations qui ne soient pas corrélées à l'exposition à l'antibiotique, dans les conditions de mesures. On obtient ainsi une série de 4 spectres de tests représentatifs de chaque concentration.

Dans cet exemple on choisit d'utiliser une méthode de classification non supervisée qui repose sur la recherche et l'utilisation d'au moins une signature spectrale caractéristique d'un effet antibiotique. Pour identifier cette signature, on utilise un jeu de données préalablement acquis pour la gentamicine et la souche EC10 dont on connait la CMI (1µg/mL) pour cet antibiotique. On utilise le jeu de données constitué par les N spectres prétraités acquis sur des bactéries exposées à l'une des concentrations (ou plusieurs) supérieure à la CMI pour extraire un effet caractéristique. On effectue une moyenne de l'ensemble des N (ou n^{∗}N) spectres du jeu de données et l'on soustrait au résultat la moyenne de N spectres acquis en l'absence d'antibiotique. Ce résultat est qualifié de signature de référence. C'est cette signature de référence qui est retenue pour qualifier les données acquises en exposant des bactéries à la ciprofloxacine, réputée inconnue dans ce cas.

Le jeu de signature ainsi construit est présenté en figure 9. Les deux signatures obtenues pour les concentrations c₁ et c₃ sont similaires : les mêmes pics sont modifiés mais l'intensité est ici corrélée à la concentration. Il est à remarquer que cette différence d'intensité pourrait servir à quantifier l'impact d'une concentration donnée mais uniquement dans certaines configurations souche/antibiotique.

On va utiliser la signature extraite à partir de la concentration 8µg/mL de gentamicine pour analyser les 3 jeux de spectres de tests. Pour ce faire on évalue la proximité de chaque spectre de tests par rapport à la signature choisie. L'évaluation de la distance entre le spectre testé et la signature sera faite dans cet exemple à l'aide d'une simple distance euclidienne dans l'espace des spectres mais de nombreuses autres distances permettent d'évaluer cette proximité (Mahalanobis, L1...). Un seuil est défini empiriquement par rapport à d'autres expériences de référence identiques réalisées précédemment, le choix de ce seuil peut-être optimisé par des méthodes classiques (ROC...) selon le niveau de résultat exigé qui peut notablement différer entre les applications (diagnostic IVD, criblage pharmaceutique...). On compare alors la mesure de distance obtenue à cette valeur de seuil pour définir la proximité entre la signature retenue et les spectres de différence acquis en présence des différentes concentrations de la molécule réputée inconnue. Si la distance est inférieure au seuil, le test est positif et l'on a détecté un effet de la molécule antibiotique. Si la distance est supérieure à ce seuil alors aucun effet n'est détecté.

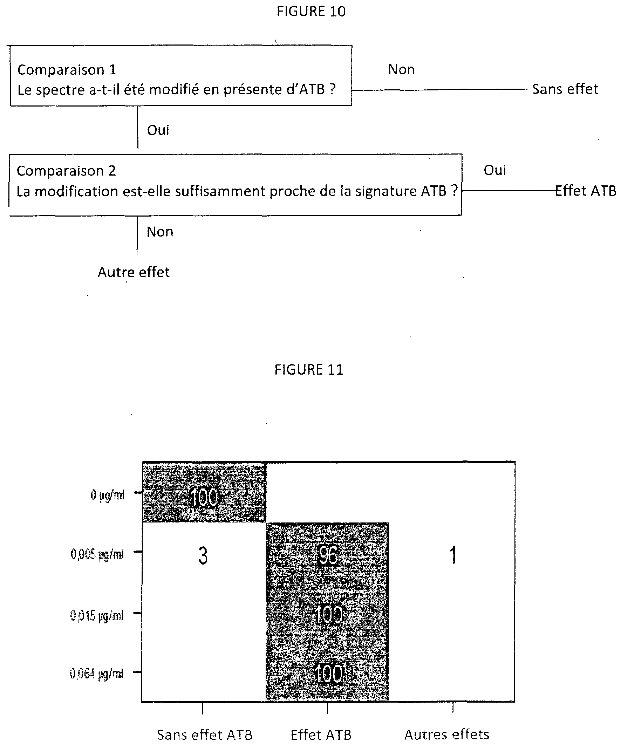

Dans un mode avantageux on peut mettre en place plusieurs seuils de détection de plus en plus stricts. Dans cet exemple, on utilise deux seuils selon ce principe : le premier, moins strict, permet de détecter une variation significative des spectres testés alors que le second, plus strict, permet de qualifier une grande proximité de la modification avec la signature. On effectue donc un test de la distance du spectre testé à la signature par rapport au premier seuil. Si le test n'est pas passé, alors aucun effet n'est détecté. Si le test est passé, la distance mesurée est inférieure au premier seuil, alors un effet est détecté. On effectue alors un second test en utilisant le second seuil et si ce test est passé, on attribue le résultat « Effet antibiotique » au test complet. Si ce second test n'est pas passé on attribue alors le résultat « Autre effet » au test complet. Cette configuration permet de détecter facilement des modifications spectrales arrivant à une concentration donnée mais ne présentant pas de similarité suffisante avec la signature de référence. Cette configuration permet donc simplement de s'affranchir d'une partie des éventuels aléas survenant pendant un test (inhomogénéité forte de la surface de capture, présence de particules parasites...). Un schéma simplifié est présenté en figure 10.

Une autre façon de réaliser un test équivalent serait d'effectuer un test en utilisant directement la norme, euclidienne ou autre, du spectre testé et de la comparer à un seuil de significativité choisi pour ne pas prendre en compte les variations classiques liées au mode de mesure (niveau de bruit du capteur, variabilité biologique,...) et d'effectuer ensuite le test à un seul seuil strict. Si la norme dépasse un certain seuil alors le spectre est significativement différent d'un spectre de différence quasi-nul si l'on n'avait pas de modifications à la concentration et l'on peut ensuite le comparerez façon stricte à la signature de référence, par exemple en mesurant sa distance à la signature au sens de la norme utilisée.

On obtient les résultats présentés dans la figure 11. On observe ainsi la détection d'un effet pour des concentrations supérieures à 0,005µg/mL. On peut ainsi donc attribuer un effet antibiotique de la ciprofloxacine sur la souche testée pour des concentrations supérieures à 0,005µg/mL. Ce test définirait donc une concentration de 0,005µg/mL comme CMI. Un nouveau test pourrait éventuellement être effectué en ajoutant de plus faibles concentrations entre 0 et 0,005µg/mL si nécessaire. Les données EUCAST pour cette souche, indique une CMI connue de 0,008µg/mL et une plage de variation acceptable de 0,004 µg/mL à 0,016 µg/mL. Un test effectué avec un etest (bioMérieux) adéquat permet de mesurer une CMI de 0,008µg/mL ce qui confirme que le résultat obtenu est conforme aux méthodes de référence [Produits bioMérieux ETEST® CI 32 (réf. 412311) et VITEK® carte N233 (réf. 413117)].

### Exemple 5 : Application du procédé de l'invention à la détermination du phénotype de sensibilité de la souche Staphylococcus aureus de référence ATCC 25923 appelée « SA44 » à l'oxacilline

Le dispositif de conditionnement retenu est constitué de deux chambres fluidiques et deux concentrations d'antibiotique sont testées: c₀ : « Sans antibiotique » et c₁ : « Test de résistance ».

Soit c₁, la concentration de l'oxacilline : c₁ = 8 µg/mL, qui correspond à multiplier par un facteur 32 la concentration 0,25µg/mL qui correspond au breakpoint clinique tel que défini par l'EUCAST. L'objectif de ce test est de déterminer si la souche bactérienne est considérée comme résistante au sens des définitions proposées par l'EUCAST.

Une solution contenant les bactéries à tester est utilisée comme échantillon de test. Cette solution est obtenue par la mise en suspension de 5.10⁷CFU/mL afin de représenter une concentration potentiellement rencontrée dans un échantillon clinique, par exemple un prélèvement urinaire. Les bactéries peuvent provenir d'une culture en milieu liquide ou d'une culture en milieu gélosé en boîte de Petri. Cette solution de bactéries est mise en contact avec l'interface **I** du dispositif fonctionnalisée par l'adsorption de polyéthylènimine (PEI) (capture générique). Après un temps de capture de 10 minutes permettant aux bactéries de rentrer en contact avec la fonctionnalisation, l'interface **I** subit un lavage avec une solution d'eau, cette étape optionnelle permettant d'éliminer le surplus de bactéries non capturées encore en solution. Le milieu physiologique retenu dans cet exemple est constitué d'un mélange peu riche de Bouillon BHI Brain Heart Infusion (par exemple de la référence 42081 de bioMérieux) et de PBS 10x (dans un rapport 1:9). Après division en deux fractions de ce milieu physiologique, on ajoute respectivement dans chacune de ces fractions une quantité d'oxacilline (par exemple de la référence O0353 de TCI Europe) permettant d'obtenir une concentration différente de gentamicine c₀ ou c₁. Les solutions de concentrations c₀ et c₁ ainsi produites sont respectivement introduites dans les chambres C₀ ou C₁. Les bactéries capturées directement à partir de l'échantillon sur la surface **I** sont ainsi exposées, dans un milieu adapté, à une concentration d'antibiotique différente selon la chambre dans laquelle elles sont présentes.

Le dispositif est ensuite chauffé pour atteindre une température de 37°C pendant deux heures puis placé en position de mesure sur le microspectromètre. Le repérage des bactéries capturées est effectué par une procédure automatique basée sur l'analyse de l'image, par une procédure classique de détection de particules, acquise au moyen de la caméra du microspectromètre et d'une source de lumière adaptée. Ce repérage permet d'acquérir automatiquement une série de spectres Raman (S₀ et S₁, respectivement) acquis sur des bactéries individuelles présentes dans chaque chambre C₀ et C₁. Le nombre de spectres à acquérir pour constituer un jeu de données dépend du niveau d'exigence sur les performances des tests à réaliser.

Comme dit précédemment, plusieurs méthodes peuvent être utilisées pour traiter les données obtenues pour aboutir au résultat. Dans le présent exemple, on utilise une méthode complète de traitement des spectres au niveau individuel comprenant une étape de prétraitement comprenant l'ensemble des phases décrites précédemment pour maximiser l'extraction d'un signal d'intérêt et la classification.

Dans cet exemple, on utilise un ensemble d'au moins 2N spectres extrait de l'ensemble M total des spectres acquis : N spectres issu de **S₀** et N spectres issus de **S₁**. Ces spectres sont tirés sans remise parmi les M spectres disponibles. On soustrait à chacun des N spectres issus de **S₁** et des N spectres issus de **S₀** la moyenne des N spectres issus de **S₀**, ces deux lots de spectres constituant un « échantillon de test de contrôle» et un « échantillon de test de résistance ».

Pour la classification, on utilise dans le présent exemple une base de données de référence obtenue à partir d'expériences similaires réalisées précédemment à des dates et à partir de cultures différentes pour entraîner un classificateur obtenu à l'aide d'un Machine à Vecteur de Support (SVM) à noyau radial. Ce classificateur est entrainé à reconnaitre deux classes, l'une « Sans effet antibiotique » à partir de spectres issus de conditions sans antibiotique et l'autre « Effet antibiotique » à partir de spectres préalablement acquis dans des conditions où la concentration est supérieure à la CMI de la ou des souches utilisées dans la base de référence. Pour chaque échantillon de tests constitués de N spectres de différence, on teste individuellement ces spectres de différence par rapport au classificateur et on attribue au groupe la classe majoritaire pour chacun des éléments des groupes. Cette attribution à la majorité est basée sur la bonne corrélation des résultats ainsi obtenus avec les méthodes de référence mais peut tout à fait être modifiée pour prendre en compte certains autres paramètres des tests. Par exemple un vote à seuil différent de la majorité où dès que le nombre de bactéries ne présentant aucun effet dépasse les 30% alors on attribue de façon conservatrice un résultat « Sans effet antibiotique ». On pourra également ajuster ce seuil pour prendre en compte le temps d'incubation : ainsi par exemple si on réduit de façon notable le temps d'exposition aux antibiotiques, ou que la bactérie testée présente un temps de doublement typique plus lent, prendre en compte un seuil plus bas pour attribuer un résultat « effet antibiotique » à ce groupe de spectres. Enfin on pourrait également adopter un système plus nuancé où chaque bactérie est considérée de façon totalement individuelle. Ce dernier mode de réalisation peut-être avantageux si la méthode de la présente invention est utilisée à des fins de recherche.

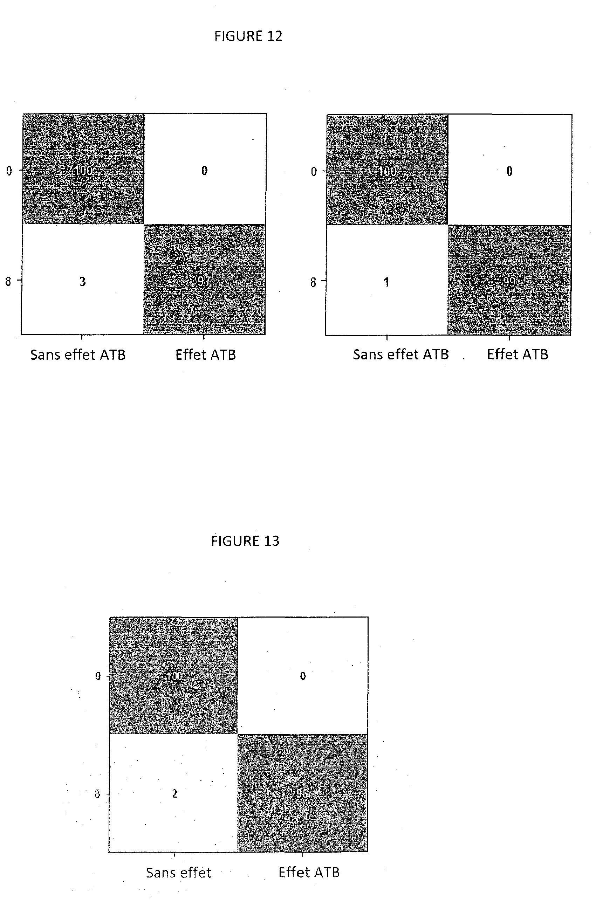

Afin d'illustrer les performances ainsi obtenues, le score moyen obtenu pour tous les résultats qui seraient obtenus avec une combinaison de 9 spectres par concentration (N=9) parmi un jeu total de spectres total acquis, 337 spectres (M=337), est présenté aux figures 12 et 13. Ces matrices présentent en colonne les états « Sans effet ATB » et « Effet ATB » et en ligne les deux concentrations d'antibiotiques testés. Le score indique le pourcentage des échantillons de test qui sont attribués à une classe donnée par le classificateur décrit précédemment. Ainsi, sur la Figure 12, on constate que 100% des échantillons de N=9 bactéries de l'« échantillon de test de contrôle» sont classés « Sans effet antibiotique » et qu'un nombre supérieur à 97% des « échantillons de test de sensibilité » constitués par des bactéries exposées à la concentration de test sont classés « Effet ATB ». La souche peut donc être qualifiée de sensible selon l'invention avec une grande confiance sur la base de seulement quelques analyses de bactéries individuelles. Les résultats observés sur ces figures sont conformes aux méthodes de référence [Produits bioMérieux ETEST® OX 256 (réf. 412432) ou VITEK® carte P631 (réf. 414961)] qui donnent pour résultat une CMI = 0,25 µg/mL ce qui confirme bien que la souche bactérienne n'est pas résistante au sens de l'EUCAST, sa CMI n'étant pas strictement supérieure au seuil défini par cet organisme.

## Revendications

1. Procédé de détermination de la réaction d'au moins une bactérie d'intérêt à son exposition à un antibiotique mettant en œuvre une analyse par spectroscopie Raman et comprenant les étapes suivantes :
On dispose d'un échantillon biologique susceptible de contenir lesdites bactéries d'intérêt,
On prépare au moins deux fractions dudit échantillon comprenant chacune une ou plusieurs bactéries d'intérêt vivantes,
On capture dans chaque fraction au moins une bactérie d'intérêt vivante à l'aide d'un partenaire de liaison,
On expose au moins l'une des fractions à au moins une concentration d'au moins un antibiotique donné, l'autre des fractions étant la fraction de contrôle,
On soumet la ou les bactérie(s) d'intérêt contenue(s)s dans les fractions à une lumière incidente et on analyse la lumière résultante obtenue par diffusion Raman par la ou les bactérie(s) d'intérêt par spectroscopie Raman pour obtenir autant de spectres Raman que de bactéries,
On traite lesdits spectres pour obtenir une signature de la réaction de la ou de chacune des bactérie(s) d'intérêt à l'exposition dudit antibiotique et du contrôle,
On compare la signature ainsi obtenue par bactérie d'intérêt, à une base de référence définie dans les mêmes conditions que ci-dessus, pour différentes bactéries et au moins ledit antibiotique, et
On définit un profil clinique de sensibilité de ladite bactérie d'intérêt audit antibiotique.

2. Procédé selon la revendication 1, **caractérisé en ce qu'**on prépare plus de deux fractions dudit échantillon et on expose au moins deux fractions à des concentrations croissantes, respectivement, dudit antibiotique.

3. Procédé selon la revendication 1 ou 2, **caractérisé en ce que** les concentrations de l'antibiotique auxquelles la ou les fractions sont respectivement exposées, sont comprises dans un intervalle de valeurs incluant au moins une valeur choisie ou des valeurs formants au moins un intervalle encadrant au moins une valeur choisie parmi des valeurs caractéristique d'un couple antibiotique/espèce tel que le cut-off épidémiologique, le ou les breakpoints clinique ou des panels de concentrations utilisés dans les méthodes de référence.

4. Procédé selon l'une quelconque des revendications 1 à 3, **caractérisé en ce que** la ou les bactérie(s) d'intérêt présentes dans les fractions sont capturées par un partenaire de liaison qui est immobilisé directement ou indirectement sur un support.

5. Procédé selon la revendication 4, **caractérisé en ce que** le partenaire de liaison est susceptible d'interagir spécifiquement avec la ou les bactérie(s) d'intérêt et est de préférence choisi parmi les protéines, les anticorps, les antigènes, les aptamères, les phages, les protéines de phages.

6. Procédé selon l'une quelconque des revendications 1 à 5, **caractérisé en ce qu'**on repère et on trie les bactéries capturées, par exemple par imagerie ou spectrophotométrie.

7. Procédé selon l'une quelconque des revendications 1 à 6, **caractérisé en ce que**, avant de soumettre la ou les bactéries d'intérêt à une lumière incidente, on élimine les bactéries n'ayant pas été capturées.

8. Procédé selon la revendication 7, **caractérisé en ce qu'**on élimine les bactéries, avant ou après l'étape d'exposition à l'antibiotique.

9. Procédé selon l'une quelconque des revendications 1 à 8, **caractérisé en ce que**, après l'étape d'exposition des fractions, on concentre lesdites fractions et la fraction de contrôle puis on les soumet à l'étape de capture.

10. Procédé selon l'une quelconque des revendications 1 à 9, **caractérisé en ce que** l'antibiotique est dans un milieu physiologique permettant au moins de maintenir la ou les bactéries d'intérêt vivantes.

11. Procédé selon l'une quelconque des revendications 1 à 10, **caractérisé en ce que** l'exposition à l'antibiotique est effectuée à une température d'au moins 18°Cet d'au plus 40°C.

12. Procédé selon l'une quelconque des revendications 1 à 11, **caractérisé en ce que** l'exposition à l'antibiotique est effectuée pendant un temps dit d'incubation d'au moins 10 minutes et d'au plus 4 heures.

13. Procédé selon l'une quelconque des revendications précédentes, **caractérisé en ce que**, pour obtenir ladite signature, pour chaque fraction exposée à l'antibiotique, on soustrait le ou les spectres Raman du prélèvement de contrôle aux spectres Raman respectivement desdits prélèvements.

14. Procédé selon l'une quelconque des revendications 1 à 13, **caractérisé en ce qu'**on détermine le statut Sensible, Intermédiaire ou Résistant de la souche d'intérêt audit antibiotique.

15. Procédé selon la revendication 14, **caractérisé en ce qu'**on détermine la concentration minimale inhibitrice (CMI) de ladite souche audit antibiotique.

16. Procédé selon la revendication 14 ou 15, **caractérisé en ce que** chaque fraction comprend au moins 2, de préférence au moins 5, bactéries d'intérêt pour obtenir au moins 2, de préférence au moins 5 signaux.

## Patentansprüche

1. Verfahren zur Bestimmung der Reaktion mindestens einer bestimmten Bakterie auf ihre Exposition gegenüber einem Antibiotikum, das eine Analyse durch Raman-Spektroskopie durchführt und die folgenden Schritte umfasst:
Verfügen über eine biologische Probe, die die bestimmten Bakterien enthalten kann,
Herstellen von mindestens zwei Fraktionen der Probe, umfassend jeweils eine oder mehrere bestimmte lebende Bakterien,
Erfassen in jeder Fraktion mindestens einer bestimmten lebenden Bakterie mit Hilfe eines Bindungspartners,
Exponieren mindestens einer der Fraktionen gegenüber mindestens einer Konzentration von mindestens einem gegebenen Antibiotikum, wobei die andere der Fraktionen die Kontrollfraktion ist,
Unterziehen der bestimmten Bakterie(n), die in den Fraktionen enthalten ist/sind, an ein einfallendes Licht und Analysieren des sich ergebenden Lichts, erhalten durch Raman-Diffusion durch die bestimmte(n) Bakterie(n) durch Raman-Spektroskopie, um ebenso viele Raman-Spektren als Bakterien zu erhalten,
Behandeln der Spektren, um eine Signatur der Reaktion der oder jeder der bestimmten Bakterie(n) beim Exponieren gegenüber dem Antibiotikum und der Kontrolle zu erhalten,
Vergleichen der so erhaltenen Signatur pro bestimmter Bakterie mit einer Referenzbasis, die durch die gleichen Bedingungen wie den oben Angegebenen definiert ist, bei verschiedenen Bakterien und mindestens dem Antibiotikum, und
Definieren eines klinischen Profils der Empfindlichkeit der bestimmten Bakterie gegenüber dem Antibiotikum.

2. Verfahren nach Anspruch 1, **dadurch gekennzeichnet, dass** mehr als zwei Fraktionen der Probe hergestellt werden und jeweils mindestens zwei Fraktionen gegenüber zunehmenden Konzentrationen des Antibiotikums exponiert werden.

3. Verfahren nach Anspruch 1 oder 2, **dadurch gekennzeichnet, dass** die Konzentrationen des Antibiotikums, gegenüber denen die Fraktion(en) jeweils exponiert wird/werden, in einem Intervall von Werten enthalten sind, darin eingeschlossen mindestens einen ausgewählten Wert oder Werten, die mindestens ein Intervall bilden, das mindestens einen Wert einschließt, der ausgewählt ist aus den Werten, die für ein Paar Antibiotikum/Art, wie z. B. den epidemiologischen Cutoff, den oder die klinischen Breakpoints oder Konzentrationspaneele, die in den Referenzverfahren verwendet werden, charakteristisch sind.

4. Verfahren nach einem der Ansprüche 1 bis 3, **dadurch gekennzeichnet, dass** die bestimmte(n) Bakterie(n), die in den Fraktionen vorhanden sind, von einem Bindungspartner erfasst werden, der direkt oder indirekt auf einem Träger blockiert wird.

5. Verfahren nach Anspruch 4, **dadurch gekennzeichnet, dass** der Bindungspartner spezifisch mit der/den bestimmten Bakterie(n) interagieren kann und vorzugsweise ausgewählt ist aus Proteinen, Antikörpern, Antigenen, Aptameren, Phagen, Phagenproteinen.

6. Verfahren nach einem der Ansprüche 1 bis 5, **dadurch gekennzeichnet, dass** die erfassten Bakterien ausfindig gemacht und sortiert werden, z. B. durch Bildgebung oder Spektrophotometrie.

7. Verfahren nach einem der Ansprüche 1 bis 6, **dadurch gekennzeichnet, dass** vor dem Unterziehen der bestimmten Bakterie(n) an ein einfallendes Licht die Bakterien, die nicht erfasst wurden, eliminiert werden.

8. Verfahren nach Anspruch 7, **dadurch gekennzeichnet, dass** die Bakterien vor oder nach dem Schritt des Aussetzens gegenüber dem Antibiotikum eliminiert werden.

9. Verfahren nach einem der Ansprüche 1 bis 8, **dadurch gekennzeichnet, dass** nach dem Schritt des Aussetzens der Fraktionen die Fraktionen und die Kontrollfraktion konzentriert werden und sie dann dem Schritt des Erfassens unterzogen werden.

10. Verfahren nach einem der Ansprüche 1 bis 9, **dadurch gekennzeichnet, dass** sich das Antibiotikum in einem physiologischen Medium befindet, das ermöglicht, mindestens die lebende(n) bestimmte(n) Bakterie(n) beizubehalten.

11. Verfahren nach einem der Ansprüche 1 bis 10, **dadurch gekennzeichnet, dass** die Exposition gegenüber dem Antibiotikum bei einer Temperatur von mindestens 18 °C und höchstens 40 °C durchgeführt wird.

12. Verfahren nach einem der Ansprüche 1 bis 11, **dadurch gekennzeichnet, dass** die Exposition gegenüber dem Antibiotikum während eines Zeitraums, bezeichnet als Inkubation, von mindestens 10 Minuten und höchstens 4 Stunden erfolgt.

13. Verfahren nach einem der vorhergehenden Ansprüche, **dadurch gekennzeichnet, dass**, um die Signatur zu erhalten, für jede gegenüber dem Antibiotikum exponierte Fraktion das oder die Raman-Spektren der Kontrollentnahme den entsprechenden Raman-Spektren der Entnahmen entzogen werden.

14. Verfahren nach einem der Ansprüche 1 bis 13, **dadurch gekennzeichnet, dass** der sensible, intermediäre oder resistente Status des bestimmten Stamms gegenüber dem Antibiotikum bestimmt wird.

15. Verfahren nach Anspruch 14, **dadurch gekennzeichnet, dass** die minimale Hemmkonzentration (CMI) des Stamms gegenüber dem Antibiotikum bestimmt wird.

16. Verfahren nach Anspruch 14 oder 15, **dadurch gekennzeichnet, dass** jede Fraktion mindestens 2, vorzugsweise mindestens 5 bestimmte Bakterien umfasst, um mindestens 2, vorzugsweise mindesten 5 Signale zu erhalten.

## Claims

1. A method for determining the reaction of at least one bacterium of interest to its exposure to an antibiotic implementing a Raman spectroscopy analysis and comprising the following steps of:
Providing a biological sample likely to contain said bacteria of interest,
Preparing at least two fractions of said sample, each comprising one or several live bacterium(bacteria) of interest,
Capturing in each fraction at least one live bacterium of interest using a binding partner,
Exposing at least one of the fractions to at least one concentration of at least one specific antibiotic, the other of the fractions being the control fraction,
Subjecting the bacterium(bacteria) of interest contained in the fractions to an incident light and analyzing the resulting light obtained by Raman scattering by the bacterium(bacteria) of interest by Raman spectroscopy to obtain as many Raman spectra as bacteria,
Processing said spectra to obtain a signature of the reaction of the or each bacterium(bacteria) of interest to the exposure of said antibiotic and the control,
Comparing the signature thus obtained for each bacterium of interest to a reference base defined under the same conditions as above, for different bacteria and at least said antibiotic, and
Defining a clinical sensitivity profile of said bacterium of interest to said antibiotic.

2. The determination method according to claim 1, **characterized in that** more than two fractions of said sample are prepared and at least two fractions are exposed to increasing concentrations, respectively, of said antibiotic.

3. The determination method according to claim 1 or 2, **characterized in that** the concentrations of the antibiotic to which the fraction(s) is/are respectively exposed, are comprised within a range of values including at least one selected value or values forming at least one interval surrounding at least one value selected from values characteristic of an antibiotic/species pair such as the epidemiological cut-off, the clinical breakpoint(s) or concentration panel(s) used in reference methods.

4. The determination method according to any one of claims 1 to 3, **characterized in that** the bacterium(bacteria) of interest present in the fractions is/are captured by a binding partner, which is directly or indirectly immobilized on a support.

5. The determination method according to claim 4, **characterized in that** the binding partner is likely to interact specifically with the bacterium(bacteria) of interest and is preferably selected from proteins, antibodies, antigens, aptamers, phages, phage proteins.

6. The determination method according to any one of claims 1 to 5, **characterized in that** the captured bacteria are identified and sorted, for example by imaging or spectrophotometry.

7. The determination method according to any one of claims 1 to 6, **characterized in that**, before subjecting the bacterium(bacteria) of interest to an incident light, the bacteria that have not been captured are removed.

8. The determination method according to claim 7, **characterized in that** the bacteria are eliminated, before or after the step of exposure to the antibiotic.

9. The determination method according to any one of claims 1 to 8, **characterized in that**, after the step of exposing the fractions, said fractions and the control fraction are concentrated then subjected to the capture step.

10. The determination method according to any one of claims 1 to 9, **characterized in that** the antibiotic is in a physiological medium allowing at least keeping the bacterium(bacteria) of interest alive.

11. The determination method according to any one of claims 1 to 10, **characterized in that** the exposure to the antibiotic is performed at a temperature of at least 18°C, at most 40°C.

12. The determination method according to any one of claims 1 to 11, **characterized in that** the exposure to the antibiotic is performed for a time called incubation time of at least 10 minutes and at most 4 hours.

13. The determination method according to any one of the preceding claims, **characterized in that**, in order to obtain said signature, for each fraction exposed to the antibiotic, the Raman spectrum(spectra) of the control collection is/are subtracted respectively from the Raman spectra of said collections.

14. The determination method according to any one of claims 1 to 13, **characterized in that** the Sensitive, Intermediate or Resistant status of the strain of interest to said antibiotic is determined.

15. The determination method according to claim 14, **characterized in that** the Minimum Inhibitory Concentration (MIC) of said strain to said antibiotic is determined.

16. The determination method according to claim 14 or 15, **characterized in that** each fraction comprises at least 2, preferably at least 5, bacteria of interest in order to obtain at least 2, preferably at least 5, signals.
